# EUROPÄISCHE PATENTSCHRIFT

(11) **EP 0 916 660 B1**
(45) Veröffentlichungstag und Bekanntmachung des Hinweises auf die Patenterteilung: **16.11.2005**
(21) Anmeldenummer: 98116653.1
(22) Anmeldetag: 03.09.1998
(51) Int. Cl.: C07D 251/46

(54) **Verfahren zur Herstellung von Sulfonylharnstoffsalzen**
Process for the production of sulphonylurea salts
Procédé de préparation de sels de sulfonylurées

(30) Priorität: 05.09.1997 CH 208497
(43) Veröffentlichungstag der Anmeldung: 19.05.1999
(73) Patentinhaber: Syngenta Participations AG, 4058 Basel (CH)
(72) Erfinder: Sting, Andrea Rolf, 5073 Gipf-Oberfrick (CH)

(56) Entgegenhaltungen:
- EP-A- 0 559 044
- DE-A- 3 609 700
- DE-A- 4 440 355
- US-A- 4 127 405
- G. E. SCHNEIDERS ET AL.: "Fate of Rimsulfuron in the Environment" J. AGRIC. FOOD CHEM., Bd. 41, Nr. 12, 1993, Seiten 2404-2410, XP002097813
- H. HÖHN ET AL.: "Potential Antidiabetic Agents. Pyrazolo[3,4-b]pyridines" J. MED. CHEM., Bd. 16, Nr. 7, 1973, Seiten 1340-1346, XP002097814
- J.-P- GIORGETTI: "Sur quelques sulfonylurées" BULL. SOC. CHIM. FR., Nr. 10, 1971, Seiten 3600-3603, XP002097815
- F. ZEUNER, H.-J. NICLAS: "N-Arylsulfonyl-N'-(pyrimidin-4-yl)ureas - Synthesis and reaction with Dimethyl Sulfoxide" J. PRAKT. CHEM., Bd. 331, Nr. 1, 1989, Seiten 121-128, XP002097816

## Beschreibung

Die vorliegende Erfindung betrifft ein Verfahren zur Herstellung von Kalium- und Natriumsalzen von N-Pyridylsulfonyl-N'-pyrimidinylharnstoffen.

Es ist bekannt, N-Pyridylsulfonyl-N'-pyrimidinylharnstoffe in der Weise herzustellen, daß man ein Pyridylsulfonamid mit einem Pyrimidinylisocyanat in Gegenwart einer Base umsetzt. Derartige Reaktionen sind beispielsweise in EP-A-0 232 067, EP-A-0 459 949 oder EP-A-0 540 697 beschrieben. Die entsprechenden Sulfonylharnstoffsalze werden erhalten, indem man die so hergestellten Sulfonylharnstoffe in einem zweiten Reaktionsschritt mit geeigneten Salzbildnern umsetzt. Solche Salzbildner sind Basen, die in der Lage sind, das saure Wasserstoffatom in der SO₂-NH-CO-Gruppierung abstrahieren zu können, wie beispielsweise Hydride, Hydroxide, Alkoholate, Hydrogencarbonate und Carbonate der Alkali- und Erdalkalimetalle. Derartige Umsetzungen sind beispielsweise in der EP-A-0 521 500 beschrieben. Dieser zweistufige Syntheseweg weist jedoch einen schwerwiegenden Nachteil auf. Das so erhaltene Sulfonylharnstoffsalz besitzt in der Regel eine nur schwach ausgeprägte Kristallstruktur und läßt sich daher nur mühsam und sehr unzureichend aus dem Reaktionsgemisch abfiltrieren. Dies verhindert insbesondere eine ökonomische großtechnische Herstellung dieser Verbindungen. Ferner können die bei den bekannten Verfahren erzielten Ausbeuten in der Regel nicht befriedigen.

Es ist daher die Aufgabe der vorliegenden Erfindung, ein Verfahren zur Herstellung von N-Pyridylsulfonyl-N'-pyrimidinylharnstoffnatrium- und kaliumsalzen bereitzustellen, welches sich durch eine einfache Reaktionsführung auszeichnet und die Nachteile der bekannten Verfahren vermeidet.

Es wurde nun gefunden, daß man N-Pyridylsulfonyl-N'-pyrimidinylharnstoffsalze auf einfache Weise und in hohen Ausbeuten herstellen kann, indem man entweder ein Pyridylsulfonamidsalz mit einem Pyrimidinylisocyanat umsetzt oder ein Pyridylsulfochlorid mit einem Pyrimidinylharnstoffsalz umsetzt.

Erfindungsgemäß wird daher vorgeschlagen, Verbindungen der Formel I 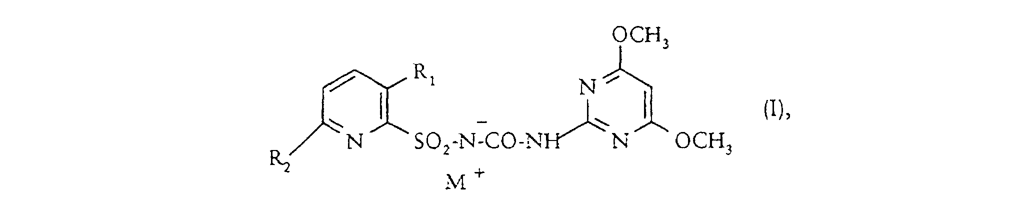 worin R₁ CO₂CH₃, CON(CH₃)₂, OCH₂CF₃, N(CH₃)COCH₃, N(CH₃)SO₂CH₃, CF₃ oder SO₂C₂H₅; R₂ Wasserstoff oder CF₃; und M Natrium oder Kalium bedeutet, in der Weise herzustellen, daß man eine Verbindung der Formel IV 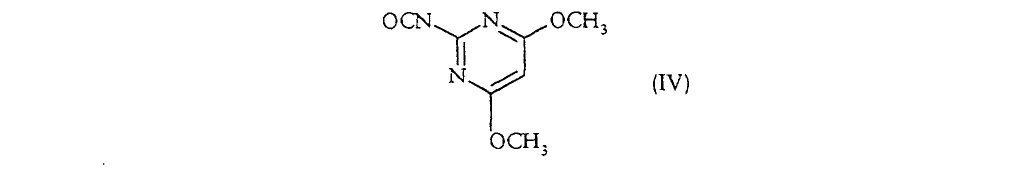 in einem aprotischen, organischen Lösungsmittel entweder mit einer Verbindung der Formel V 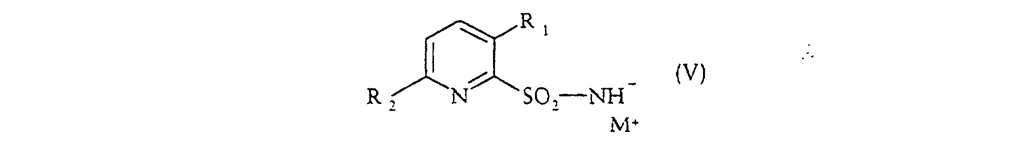 worin R₁, R₂ und M die unter Formel I angegebenen Bedeutungen haben, umsetzt, oder mit NH₃ in einem aprotischen, organischen Lösungsmittel in die Verbindung der Formel III 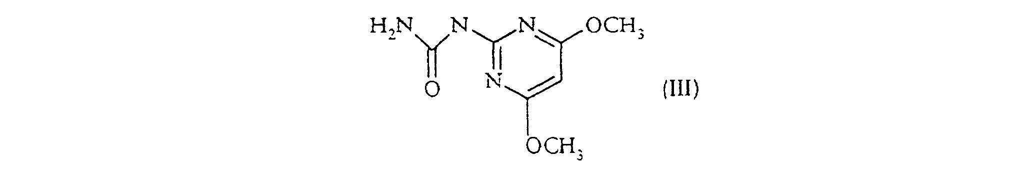 überführt, diese mit einem Hydrid, Hydroxid, Alkoholat, Hydrogencarbonat oder Carbonat von Natrium oder Kalium in einem aprotischen, organischen Lösungsmittel zur Verbindung der Formel II 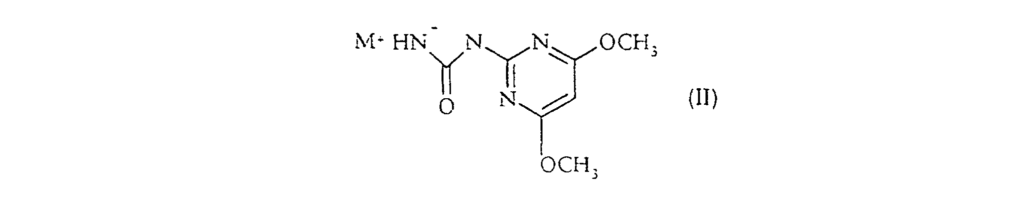 worin M die unter Formel I angegebene Bedeutung hat, umsetzt, und diese anschließend mit einer Verbindung der Formel VI 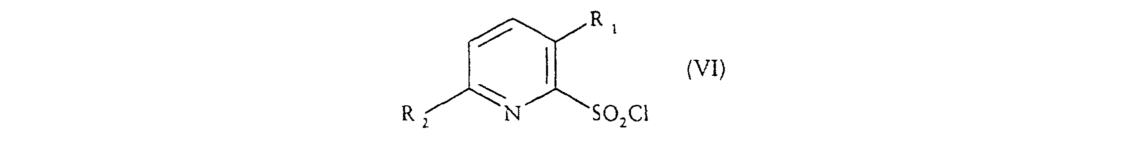 worin R₁ und R₂ die unter Formel I angegebene Bedeutung haben, umsetzt.

Für die Umsetzung der Verbindung der Formel IV mit der Verbindung der Formel V geeignete aprotische, organische Lösungsmittel sind beispielsweise Essigsäureethylester, Acetonitril, Dimethylsulfoxid, Dimethylformamid, Dimethylacetamid, N-Methyl-2-pyrrolidon, Aceton, Butanon, halogenierte Lösungsmittel wie beispielsweise Methylenchlorid, Trichlormethan oder Trichlorethan, Ether wie Tetrahydrofuran, Diethylether, 1,2-Dimethoxyethan, Dioxan, Methyl-tert.-Butylether, ferner aromatische Lösungsmittel wie Chlorbenzol, Toluol und Xylol. Besonders bevorzugt ist Dioxan und Tetrahydrofuran. Die Umsetzung der Verbindung der Formel IV mit der Verbindung der Formel V wird bei Temperaturen von - 20 °C bis 180 °C durchgeführt, wobei ein Temperaturbereich von 30 - 80 °C bevorzugt ist. Die Verbindungen der Formeln IV und V können in äquivalenten stöchiometrischen Mengen eingesetzt werden, wobei aber ein geringer Überschuß an Isocyanat von Vorteil sein kann.

Für die Umsetzung der Verbindung der Formel IV zur Verbindung der Formel III geeignete aprotische, organische Lösungsmittel sind beispielsweise Essigsäureethylester, Acetonitril, Dimethylsulfoxid, Dimethylformamid, Dimethylacetamid, N-Methyl-2-pyrrolidon, Aceton, Butanon, halogenierte Lösungsmittel wie beispielsweise Methylenchlorid, Trichlormethan oder Trichlorethan, Ether wie Tetrahydrofuran, Diethylether, 1,2-Dimethoxyethan,Dioxan, Methyl-tert.-Butylether, ferner aromatische Lösungsmittel wie Chlorbenzol, Toluol und Nylol. Besonders bevorzugt ist Dioxan und Tetrahydrofuran. Die Umsetzung wird bei Temperaturen von - 20 °C bis 180 °C durchgeführt, wobei ein Temperaturbereich von 0 - 80 °C bevorzugt ist. Die Verbindung der Formel IV wird in einem stöchiometrischen Verhältnis zum eingesetzten NH₃ von 1 : 1 bis 1 : 3, vorzugsweise 1 : 1,5 eingesetzt.

Für die Überführung der Verbindung der Formel III in die Verbindung der Formel II geeignete aprotische, organische Lösungsmittel sind beispielsweise Essigsäureethylester, Acetonitril, Dimethylsulfoxid, Dimethylformamid, Dimethylacetamid, N-Methyl-2-pyrrolidon, Aceton, Butanon, halogenierte Lösungsmittel wie beispielsweise Methylenchlorid, Trichlormethan oder Trichlorethan, Ether wie Tetrahydrofuran, Diethylether, 1,2-Dimethoxyethan, Dioxan, Methyl-tert.-Butylether, ferner aromatische Lösungsmittel wie Chlorbenzol, Toluol und Xylol sowie Alkohole wie C₁-C₅-Alkohole wie Methanol oder Ethanol. Besonders bevorzugt ist Tetrahydrofuran.

Für die Umsetzung der Verbindung der Formel II mit der Verbindung der Formel VI geeignete aprotische, organische Lösungsmittel sind beispielsweise Essigsäureethylester, Acetonitril, Dimethylsulfoxid, Dimethylformamid, Dimethylacetamid, N-Methyl-2-pyrrolidon, Aceton, Butanon, halogenierte Lösungsmittel wie beispielsweise Methylenchlorid, Trichlormethan oder Trichlorethan, Ether wie Tetrahydrofuran, Diethylether, 1,2-Dimethoxyethan, Dioxan, Methyl-tert.-Butylether, ferner aromatische Lösungsmittel wie Chlorbenzol, Toluol und Xylol. Besonders bevorzugt ist Tetrahydrofuran. Die Umsetzung wird bei Temperaturen von - 20 °C bis 180 °C durchgeführt, wobei ein Temperaturbereich von 0 - 40 °C bevorzugt ist. Das stöchiometrische Verhältnis zwischen der Verbindung der Formel III, der Base und der Verbindung der Formel VI beträgt 1 : 2 : 1 bis 1 : 3 : 2, vorzugsweise 1 : 2 : 1,1. Für die Salzbildung geeignete Basen sind beispielsweise die Hydride, Carbonate, Hydrogencarbonate und Alkoholate des Natriums und Kaliums, vorzugsweise die Hydride und Alkoholate wie Natriumhydrid, Kaliumhydrid, Natriummethylat, Kaliummethylat oder Kaliumethylat, besonders bevorzugt Natriumhydrid und Natriummethylat.

Vorzugsweise setzt man im erfindungsgemäßen Verfahren eine Verbindung der Formel IV 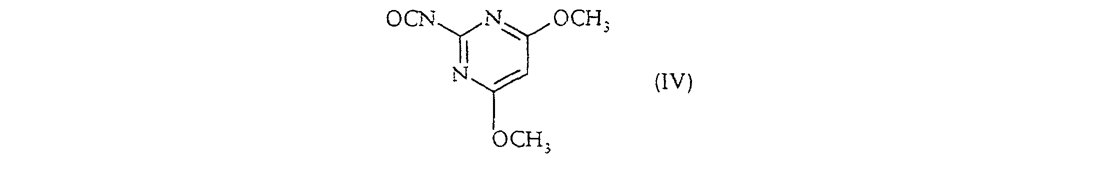 in einem aprotischen organischen Lösungsmittel mit einer Verbindung der Formel V 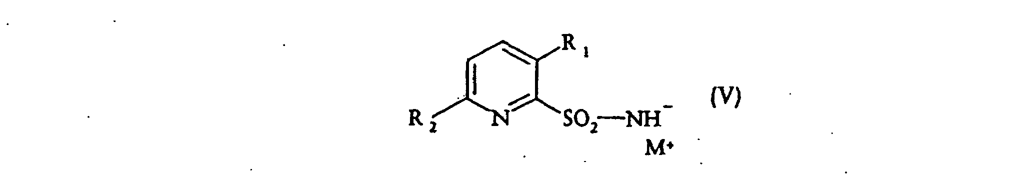 worin R₁, R₂ und M die unter Formel I angegebenen Bedeutungen haben, um. Bei dieser Variante ist insbesondere die überraschend hohe Ausbeute hervorzuheben.

Besonders bevorzugt werden diejenigen Verbindungen der Formel I nach dem erfindungsgemäßen Verfahren hergestellt, worin R₁ OCH₂CF₃ und R₂ Wasserstoff bedeuten. In einer weiteren Gruppe von bevorzugten Verbindungen der Formel I steht M für Natrium.

In einer bevorzugten Variante des erfindungsgemäßen Verfahrens setzt man 3-(2-Trifluorethoxy)-pyridin-2-ylsulfonamid-Natriumsalz bei einer Temperatur von 30 bis 80 °C in Dioxan oder Tetrahydrofuran mit 4,6-Dimethoxy-pyrimidin-2-isocyanat um.

Das Zwischenprodukt der Formel II 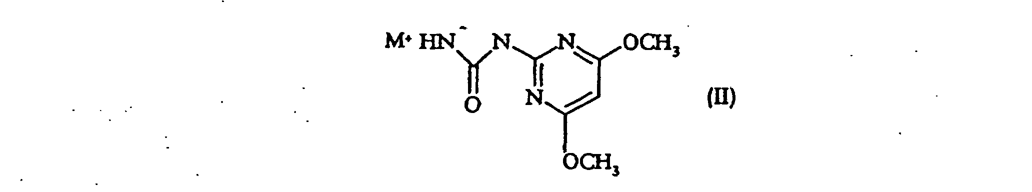 worin M die unter Formel I angegebene Bedeutung hat, ist neu, wurde speziell für das erfindungsgemäße Verfahren entwickelt und bildet daher ebenfalls einen Gegenstand der vorliegenden Erfindung.

Die Verbindungen der Formel I bzw. die entsprechenden Sulfonylharnstoffe sind bekannt und beispielsweise in EP-A-0 459 949, EP-A-0 540 697, EP-A-0 103 543, EP-A-0 600 836 und

EP-A-0 521 500 beschrieben. Die Sulfonylharnstoffsalze der Formel I können in amorpher Form sowie als Solvate, beispielsweise mit Dioxan, oder als Hydrate, beispielsweise als Mono- und Dihydrate, vorliegen.

Die Herstellung der Ausgangsverbindung der Formel IV ist beispielsweise in EP-A-0 232 067, Seite 29 beschrieben. Die Verbindungen der Formel V lassen sich beispielsweise herstellen, indem man die Verbindung der Formel VII 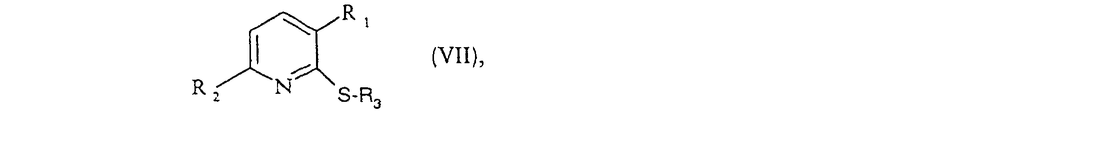 worin R₁ und R₂ die unter Formel I angegebene Bedeutungen haben und R₃ für -CH₂-Phenyl oder Isopropyl steht, durch wäßrige Chlorierung in die Verbindung der Formel VI überführt. Diese wird mit wäßrigem Ammoniak behandelt und das entstandende Sulfonamid anschließend mit 30 %igem Natriummethylat umgesetzt. Derartige Reaktionen sind bekannt und dem Fachmann geläufig.

Die folgenden Beispiele erläutern das erfindungsgemäße Verfahren weiter.

### Herstellungsbeispiele:

### Beispiel H1: Herstellung von N-[3-(2-Trifluorethoxy)-pyridin-2-yl-sulfonyl]-N'-(4,6-dimethoxy-pyrimidin-2-yl)-harnstoff-Natritumsalz:

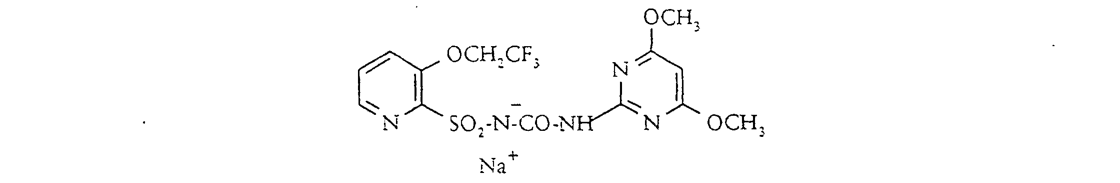

Zu einer Suspension aus 35,9 g (o,129 mol) 3-(2-Trifluorethoxy)-pyridin-2-ylsulfonamid-Natriumsalz in 200 g Dioxan gibt man bei einer Temperatur von 50 °C innerhalb von 30 Minuten eine Lösung aus 23.3 g (0.129 mol) 4,6-Dimedioxy-pyrimidin-2-isocyanat in 720 g Dioxan tropfenweise hinzu. Anschließend wird das Reaktionsgemisch eine Stunde lang bei einer Temperatur von 50 °C gehalten. Nach dem Abkühlen wird das Gemisch mit einem Saugfilter filtriert, der erhaltene Rückstand mit 100 g Dioxan gewaschen und danach bei bei einer Temperatur von 100 °C im Trockenschrank getrocknet. Man erhält 57.16 g N-[3-(2-Trifluorethoxy)-pyridin-2-yl-sulfonyl]-N'-(4,6-dimethoxy-pyrimidin-2-yl)-harnstoff-Natriumsalz (Gehalt: 95 %, Ausbeute: 92 % der Theorie).

Vergleichbare Ergebnisse erhält man wenn man die Reaktion in Tetrahydrofuran als Lösungsmittel durchführt.

### Beispiel H2: Herstellung von N-[3-(2-Trifluorethoxy)-pyridin-2-yl-sulfonyl]-N'-(4,6-dimethoxy-pyrimidin-2-yl)-harnstoff-Natriumsalz:

a) In eine Lösung aus 23,3 g (0,129 mol) 4,6-Dimethoxy-pyrimidin-2-isocyanat in 560 g Dioxan leitet man innerhalb von 60 Minuten bei einer Temperatur von 10 bis 15 °C 3.29 g (0.194mol) NH₃-Gas ein. Nach Einengen der Lösung erhält man 27 g 2-Aminocarbonylamino-4,6-Dimethoxy-pyrimidin (Gehalt: 87 %, Ausbeute: 92 % der Theorie).
b) Zu einer Suspension von 0,87 g (Gehalt: 50%, 0,02 mol) Natriumhydrid in 50 ml Tetrahydrofuran gibt man nach Kühlung auf eine Temperatur von 0 bis -5 °C eine Lösung aus 2,2 g (0,01 mol) 2-Aminocarbonylamino-4,6-dimethoxy-pyrimidin in 50 ml Tetrahydrofuran tropfenweise hinzu. Nach Entfernen der Kühlung läßt man die Temperatur auf 20 °C ansteigen und wartet, bis die Gasentwicklung abgeklungen ist. Anschließend gibt man eine Lösung aus 3 g (0,011 mol) 3-(2-Trifluorethoxy)-pyridin-2-yl-sulfonchlorid in 10 ml Tetrahydrofuran innerhalb von 10 Minuten tropfenweise hinzu und rührt die Reaktionsmischung eine Stunde lang. Anschließend destilliert man bei einem Druck von 30 kPa und einer Temperatur von 32°C 60 ml Tetrahydrofuran bis zur Eintrübung der Lösung ab. Nach Entfernen des Heizbades und Rühren der Mischung für 12 Stunden bei einer Temperatur von 20 °C kristallisiert das Produkt aus. Man kühlt auf eine Temperatur von 0 °C und filtriert nach 30 Minuten mit einem Saugfilter. Der erhaltene Rückstand wird mit 15 ml Tetrahydrofuran gewaschen und bei einer Temperatur von 100 °C getrocknet. Man erhalt 2,8 g N-[3-(2-Trifluorethoxy)-pyridin-2-yl-sulfonyl]-N'-(4,6-dimethoxy-pyrimidin-2-yl)-harnstoff-Natriumsalz mit einem Gehalt von 97% und einer Ausbeute von 59% d.Th..

Mit dem Verfahren gemäß vorliegender Erfindung ist es möglich, N-Pyridylsulfonyl-N'-pyrimidinylharnstoffnatrium- und kaliumsalze auf einfache Weise und in hohen Ausbeuten herzustellen. Gegenüber den aus den bekannten Verfahren erhaltenen Salzen besitzt das gemäß vorliegender Erfindung hergestellte Sulfonylharnstoffsalz unerwarteterweise eine wesentlich ausgeprägtere kristalline Struktur und läßt sich daher auf einfache Weise mit deutlich erhöhter Geschwindigkeit aus der Reaktionsmischung abtrennen. Ferner werden insbesondere bei der Umsetzung der Verbindung der Formel IV mit der Verbindung der Formel V überraschend hohe Ausbeuten von über 90 % d.Th. bei einem Umsatz von bis zu 99,5 % erzielt, was mit den bekannten Verfahren nicht möglich war.

## Patentansprüche

1. Verfahren zur Herstellung von Verbindungen der Formel I, 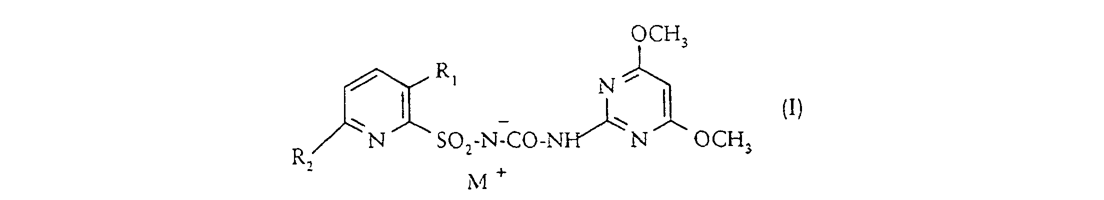 worin R₁ CO₂CH₃, CON(CH₃)₂, OCH₂CF₃, N(CH₃)COCH₃, N(CH₃)SO₂CH₃, CF₃ oder SO₂C₂H₅; R₂ Wasserstoff oder CF₃; und M Natrium oder Kalium bedeutet, **dadurch gekennzeichnet, daß** man eine Verbindung der Formel IV 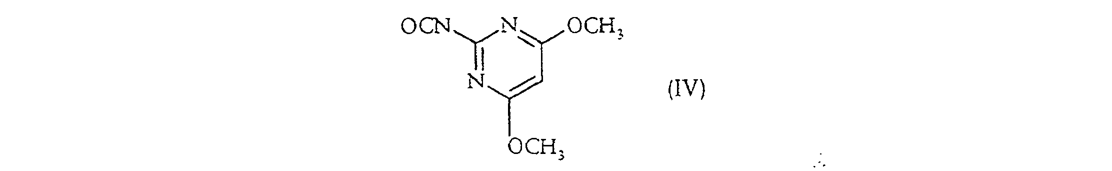 in einem aprotischen organischen Lösungsmittel entweder mit einer Verbindung der Formel V 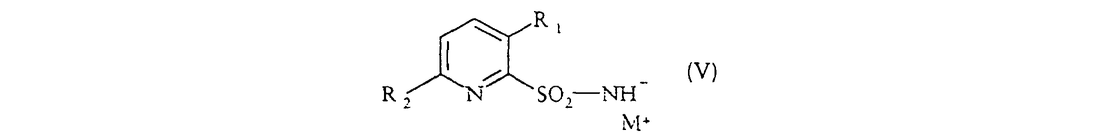 worin R₁, R₂ und M die unter Formel I angegebenen Bedeutungen haben, umsetzt, oder mit NH₃ in einem aprotischen organischen Lösungsmittel in die Verbindung der Formel III 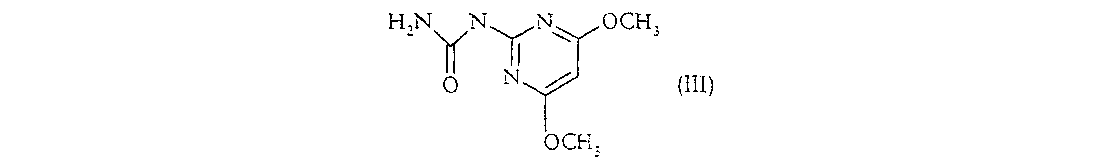 überführt, diese mit einem Hydrid, Hydroxid, Alkoholat, Hydrogencarbonat oder Carbonat von Natrium oder Kalium in einem aprotischen organischen Lösungsmittel zur Verbindung der Formel II 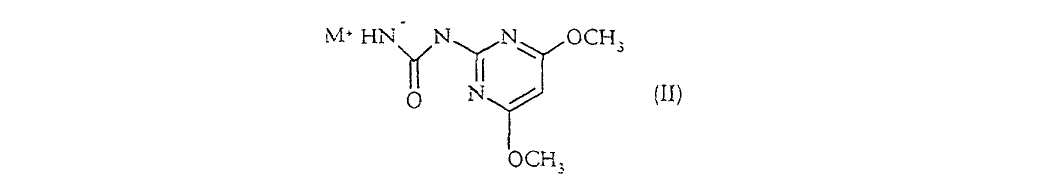 worin M die unter Formel I angegebene Bedeutung hat, umsetzt, und diese anschließend mit einer Verbindung der Formel VI 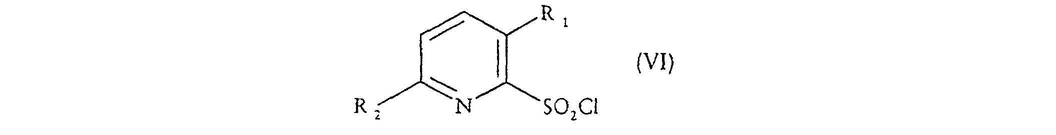 worin R₁ und R₂ die unter Formel I angegebene Bedeutung haben, umsetzt.

2. Verfahren nach Anspruch 1, **dadurch gekennzeichnet, daß** man eine Verbindung der Formel IV 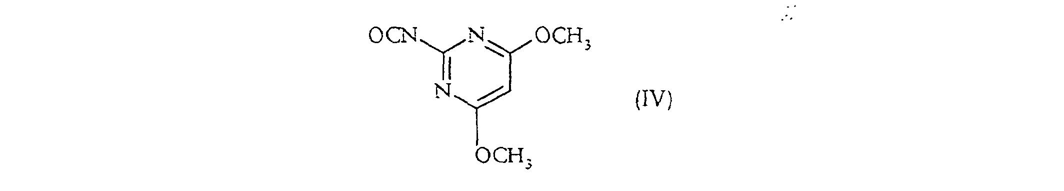 in einem aprotischen organischen Lösungsmittel mit einer Verbindung der Formel V 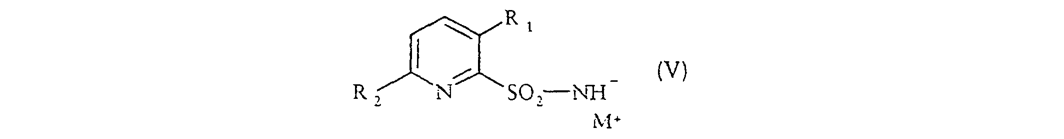 worin R₁,R₂ und M die unter Formel I angegebenen Bedeutungen haben, umsetzt.

3. Verfahren nach Anspruch 1, **dadurch gekennzeichnet, daß** R₁ OCH₂CF₃ und R₂ Wasserstoff bedeuten.

4. Verfahren nach Anspruch 1, **dadurch gekennzeichnet, daß** M für Natrium steht.

5. Verfahren nach Anspruch 1, **dadurch gekennzeichnet, daß** man 3-(2-Trifluorethoxy)-pyridin-2-ylsulfonamid-Natriumsalz bei einer Temperatur von 30 bis 80 °C in Dioxan oder Tetrahydrofuran mit 4,6-Dimethoxy-pyrimidin-2-isocyanat umsetzt.

6. Verbindungen der Formel II 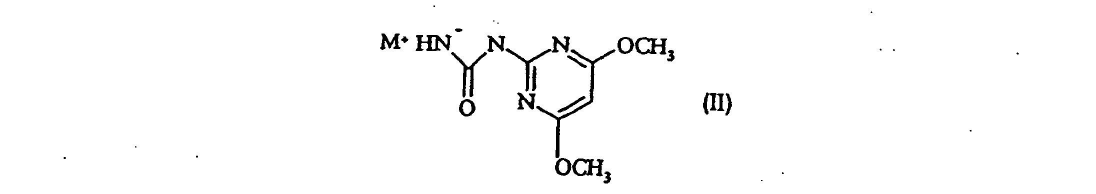 worin M die unter Formel I angegebene Bedeutung hat.

7. Verwendung der Verbindung der Formel III nach Anspruch 1, zur Herstellung von Verbindungen der Formel I

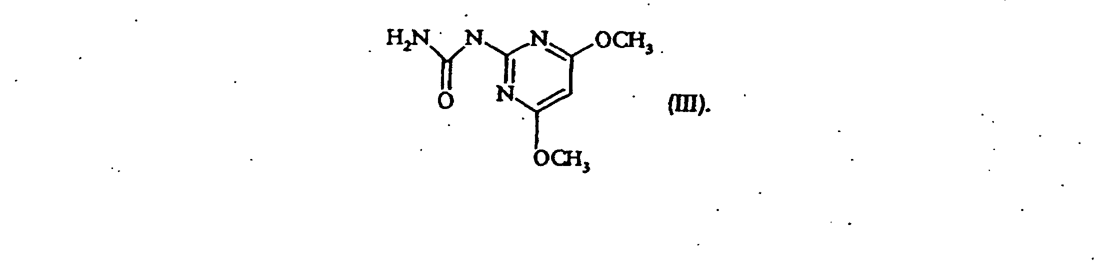

## Claims

1. A process for the preparation of a compound of formula I 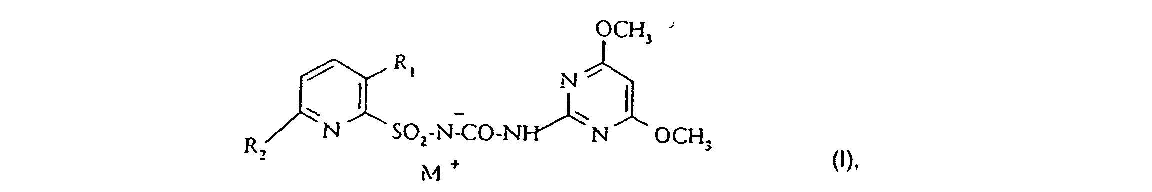 wherein R₁ is CO₂CH₃, CON(CH₃)₂, OCH₂CF₃, N(CH₃)COCH₃, N(CH₃)SO₂CH₃, CF₃ or SO₂C₂H₅; R₂ is hydrogen or CF₃; and M is sodium or potassium, which comprises either reacting a compound of formula IV 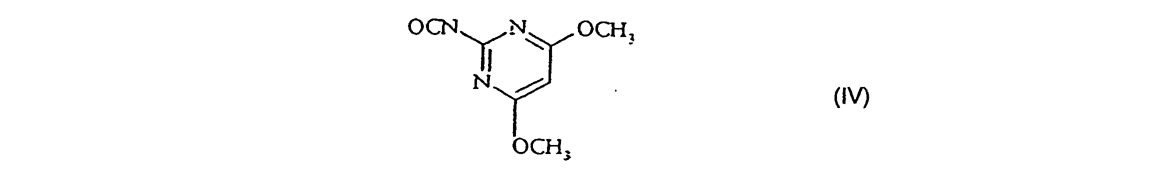 in an aprotic organic solvent with a compound of formula V 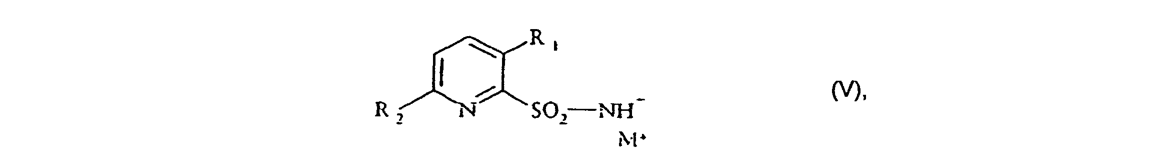 wherein R₁, R₂ and M are as defined for formula I, or converting a compound of formula IV in an aprotic organic solvent using NH₃ to form the compound of formula III 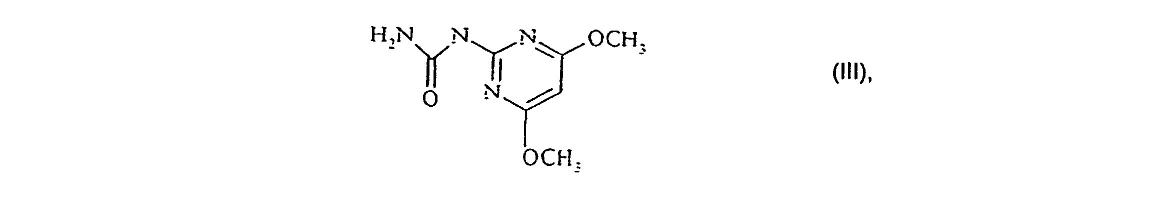 reacting that compound with a hydride, hydroxide, alcoholate, hydrogen carbonate or carbonate of sodium or of potassium in an aprotic organic solvent to form the compound of formula II 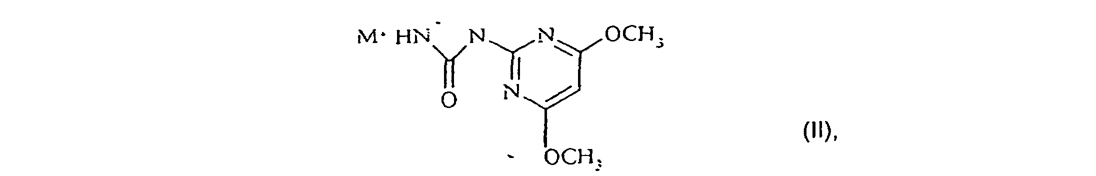 wherein M is as defined for formula I, and then reacting that compound with a compound of formula VI 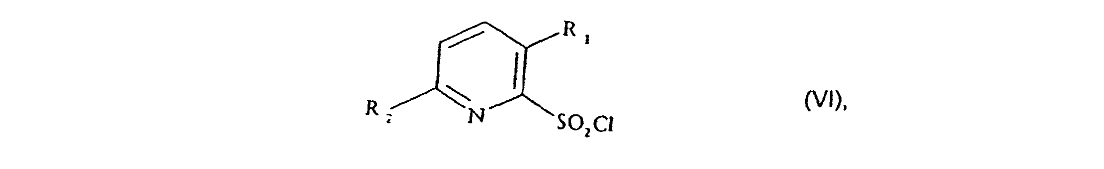 wherein R₁ and R₂ are as defined for formula I.

2. A process according to claim 1, which comprises reacting a compound of formula IV 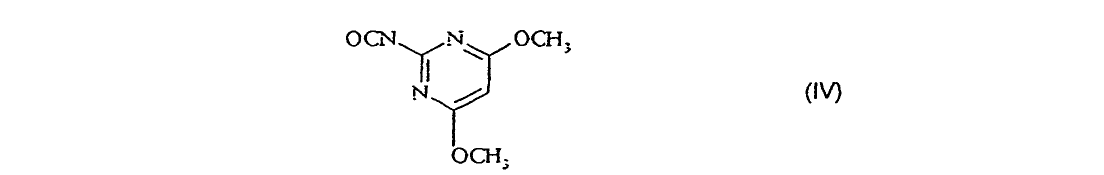 in an aprotic organic solvent with a compound of formula V 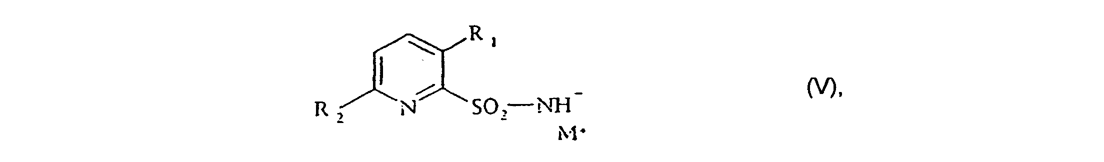 wherein R₁, R₂ and M are as defined for formula I.

3. A process according to claim 1, wherein R₁ is OCH₂CF₃ and R₂ is hydrogen.

4. A process according to claim 1, wherein M is sodium.

5. A process according to claim 1, which comprises reacting 3-(2-trifluoroethoxy)pyrid-2-ylsulfonamide sodium salt with 4,6-dimethoxypyrimidine 2-isocyanate at a temperature of from 30 to 80°C in dioxane or tetrahydrofuran.

6. A compound of formula II 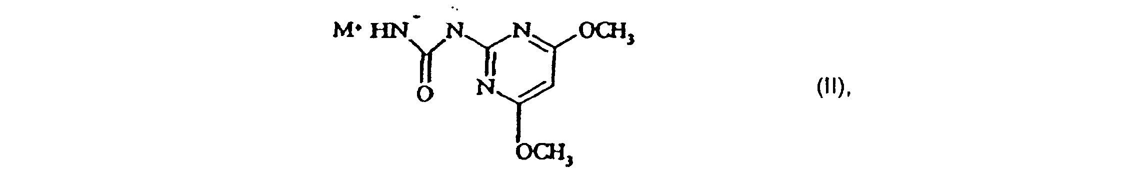 wherein M is as defined for formula I.

7. The use of the compound of formula III according to claim 1 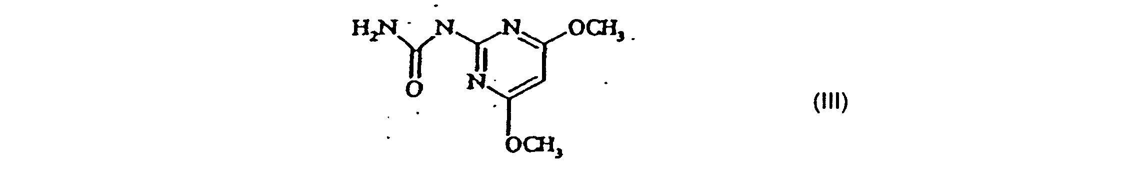 in the preparation of a compound of formula I.

## Revendications

1. Procédé pour la préparation de composés de formule I 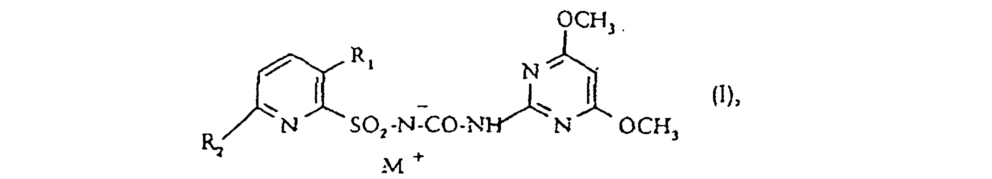 dans laquelle R₁ représente CO₂CH₃, CON(CH₃)₂, OCH₂CF₃, N(CH₃)COCH₃, N(CH₃)SO₂CH₃, CF₃ ou SO₂C₂H₅ ; R₂ représente un atome d'hydrogène ou CF₃ ; et M représente le sodium ou le potassium, **caractérisé en ce que** on fait réagir un composé de formule IV 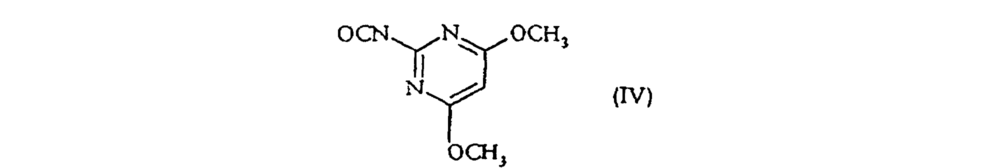 dans un solvant organique aprotique soit avec un composé de formule V 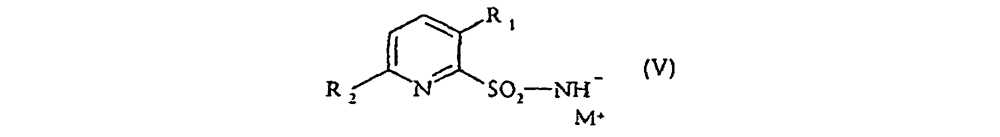 dans laquelle R₁, R₂ et M ont les significations données sous la formule I, soit on le convertit, avec NH₃ dans un solvant organique aprotique, en le composé de formule III 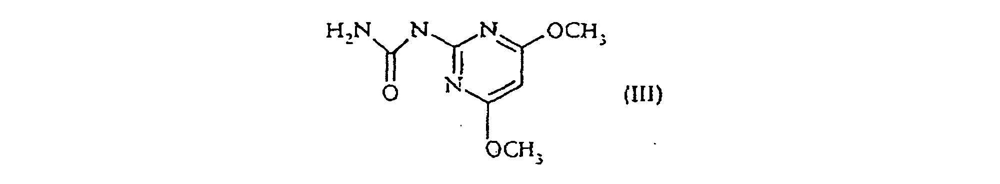 puis on fait réagir ce dernier avec un hydrure, hydroxyde, alcoolate, hydrogénocarbonate ou carbonate de sodium ou de potassium, dans un solvant organique aprotique, pour aboutir au composé de formule II 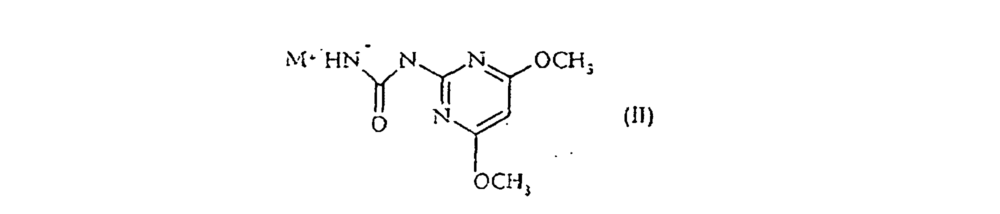 dans laquelle M a la signification donnée sous la formule I, et on fait ensuite réagir celui-ci avec un composé de formule VI 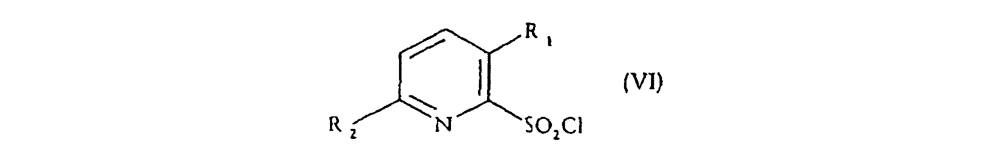 dans laquelle R₁ et R₂ ont les significations données sous la formule I.

2. Procédé selon la revendication 1, **caractérisé en ce qu'**on fait réagir un composé de formule IV 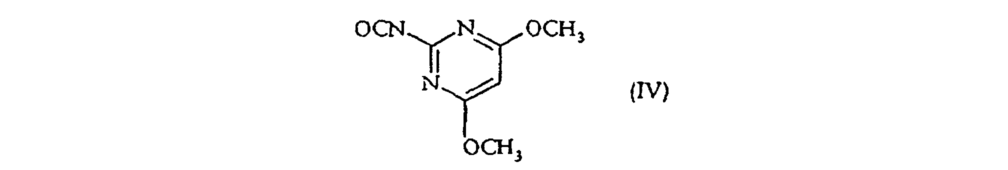 dans un solvant organique aprotique, avec un composé de formule V 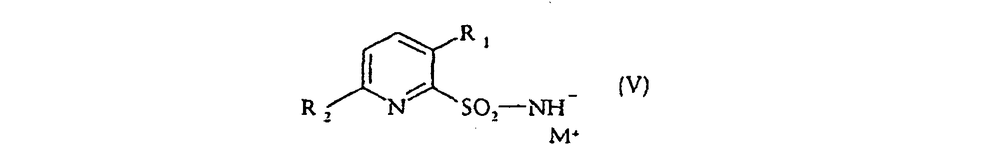 dans laquelle R₁, R₂ et M ont les significations données sous la formule I.

3. Procédé selon la revendication 1, **caractérisé en ce que** R₁ représente OCH₂CF₃ et R₂ représente un atome d'hydrogène.

4. Procédé selon la revendication 1, **caractérisé en ce que** M représente le sodium.

5. Procédé selon la revendication 1, **caractérisé en ce qu'**on fait réagir du sel sodique de 3-(2-trifluoroéthoxy)pyridin-2-yl-sulfonamide à une température de 30 à 80°C dans du dioxanne ou du tétrahydrofuranne, avec du 4,6-diméthoxypyrimidine-2-isocyanate.

6. Composés de formule II 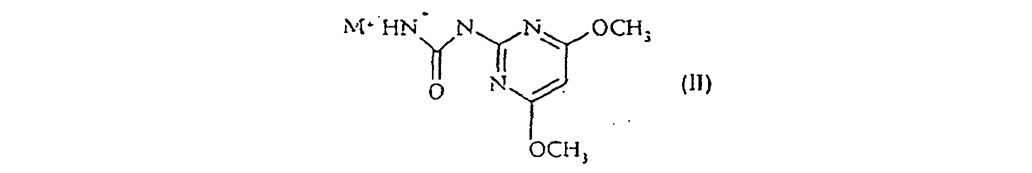 dans laquelle M a la signification donnée sous la formule I.

7. Utilisation du composé de formule III selon la revendication 1, pour la préparation de composés de formule I
